# EUROPÄISCHE PATENTSCHRIFT

(11) **EP 1 034 010 B1**
(45) Veröffentlichungstag und Bekanntmachung des Hinweises auf die Patenterteilung: **02.05.2007**
(21) Anmeldenummer: 98951495.5
(22) Anmeldetag: 05.10.1998
(51) Int. Cl.: A61M 25/01

(54) **ENDOSKOPISCHE EINFÜHRHILFSVORRICHTUNG**
ENDOSCOPIC INSERTION AID
DISPOSITIF D'AIDE A L'INSERTION ENDOSCOPIQUE

(30) Priorität: 03.10.1997 LV 970190; 23.09.1998 LV 980188
(43) Veröffentlichungstag der Anmeldung: 13.09.2000
(73) Patentinhaber: Karl Storz GmbH & Co. KG, 78532 Tuttlingen (DE); Matasov, Sergej, 1048 Riga (LV)
(72) Erfinder: MATASOV, Sergej, LV-1048 Riga (LV)
(74) Vertreter: Eder, Thomas
(86) Internationale Anmeldenummer: PCT/EP1998/006338
(87) Internationale Veröffentlichungsnummer: WO 1999/017828

(56) Entgegenhaltungen:
- EP-A1- 0 541 258
- DE-C1- 3 329 176

## Beschreibung

Die Erfindung betrifft ein Endoskop, insbesondere eine Einführhilfsvorrichtung für ein Endoskop, welches auf dem Gebiet der Medizin, insbesondere der transanalen Kolonoskopie und Enteroskopie, einsetzbar ist. Die Erfindung kann aber auch in industriellen Endoskopen zur Anwendung kommen.

Aus der DE 3 329 176 ist medizinisches Gerät zum Untersuchen oder Behandeln von Körperhöhlen bekannt, welches eine Einführungshilfsvorrichtung nach dem Prinzip des sich ausstülpenden Schlauchgebildes umfasst. Die Einführungshilfsvorrichtung besteht aus einem beidseitig offenen Rohr mit einem seitlichen Druckanschlussstutzen und einem durch das Rohr laufenden flexiblen ausstülpbaren Schlauchgebilde, dessen beide Enden mit jeweils einem Ende des Rohrs verbunden sind. Das medizinische Gerät läuft innerhalb des Schlauchgebildes durch das Rohr. Am distalen Ende des medizinischen Geräts ist das Schlauchgebilde in mehreren in axialer Richtung relativ langen Doppellagen aufgefaltet, welche parallel zum einzuführenden Röhrchen verlaufen. Zu den Nachteilen der genannten Vorrichtung gehört der nicht aufeinanderfolgende Abzug des schlauchartigen Gebildes der Einführhilfsvorrichtung, der durch das "Zusammenkleben" der Lagen unter Einwirkung von Luftdruck und das unvermeidbare Eindringen von Luft in die Räume zwischen den Lagen des Schlauchgebildes verursacht wird. Das vorzeitige Herausschieben einer Lage schließt das Mitwirken anderer Lagen, die über der herausgedrehten Lage liegen, an der Intubation aus.

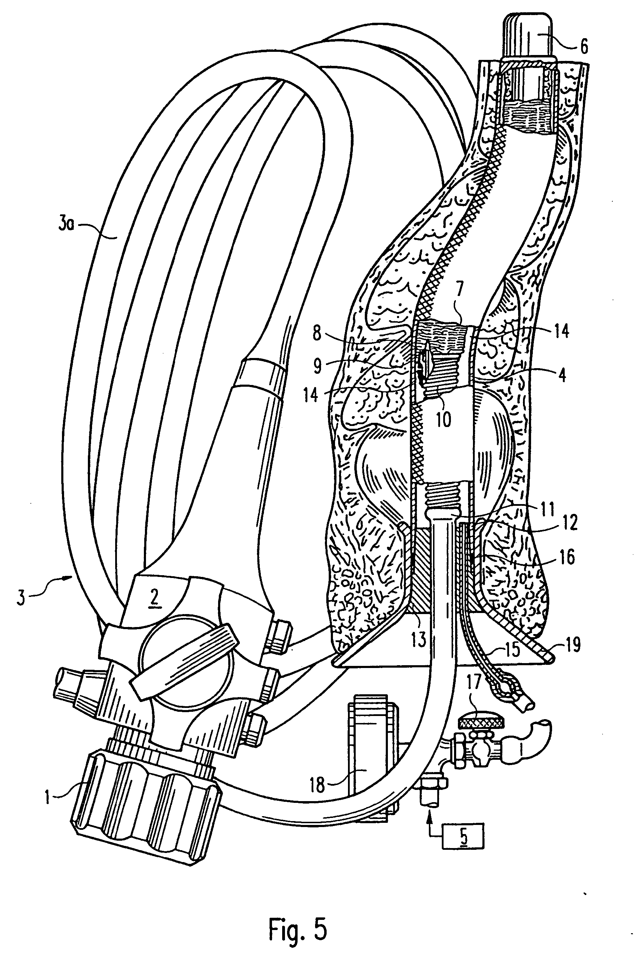

Aus der SU 1 522 466 ist eine Darmendoskop-Einrichtung mit einer Einführhilfsvorrichtung mit einem Schlauchgebilde mit kurzen Lagen bekannt, die im rechten Winkel zum durch das Endoskop einzuführenden endoskopischen Röhrchen angeordnet sind. Diese Endoskop-Einrichtung ist als der nächste Stand der Technik zur vorliegenden Erfindung anzusehen. Die Endoskop-Einrichtung gemäß der SU 1. 522 466 umfasst, wie aus Fig. 5 ersichtlich, eine Lichtquelle, eine Überdruckquelle 5 und ein Endoskop 3 mit einem Okularglas 1, einem Pult 2, das über eine Kommunikationsabzweigung verfügt, sowie einem Anschlag oder einer Stütze 11 für eine Feder 10. Des Weiteren umfasst diese Endoskop-Einrichtung eine am Endoskop 3 vorgesehene Einführhilfsvorrichtung, die einen (bereits im Ausgangszustand) nach außen gestülpten Teil 4 und einen in das Teil 4 eingeschlossenen (im Ausgangszustand) nicht nach außen gestülpten Teil eines ausstülpbaren Schlauchgebildes aufweist, wobei das nicht nach außen gestülpte Teil des ausstülpbaren Schlauchgebildes spielfrei am Endoskoprohr anliegt und kurze gefaltete Lagen aufweist, die im Wesentlichen rechtwinklig zur Achse des Endoskoprohres verlaufen.

Das Ende 7 des nicht nach außen gestülpten bzw. gefalteten Teils des Schlauchgebildes wird durch eine Feder 10 gestützt bzw. beaufschlagt, und der Übergang des nicht nach außen gestülpten Teils in den nach außen gestülpten Teil 4 des Schlauchgebildes, d.h. das "Ausstülpende", ist durch ein Endstück 6 begrenzt bzw. liegt an dessen rückwärtigen Ende an.

Außerdem umfaßt die Endoskop-Einrichtung gemäß der SU 1 522 466 eine in einem Analdilatator 19 vorgesehene äußere flexible Dichtung 13 des Endoskops 3, auf der das Ende 12 des nach außen gestülpten Teils 4 des Schlauchgebildes durch einen Ring 16 fixiert ist, sowie Ringe 8, 9 auf dem nicht nach außen gestülpten inneren Ende 7 des ausstülpbaren Schlauchgebildes. Es ist weiterhin eine Luftleitung 15 mit einem Hahn 17 vorgesehen, durch die der Arbeitsdruck in den Raum 14 des nach außen gestülpten Teils 4 des Schlauchgebildes der Einführhilfsvorrichtung gelangt.

Außer Vorrichtungen zur Licht- und Bildübertragung, Biopsiekanälen, Zugabe von Gas oder Flüssigkeit umfaßt die Endoskop-Einrichtung gemäß der SU 1 522 466 zwei Paare eng gewickelter Federn, die Züge einschließen und die paarweise einen Distalring eines Mechanismus zum Biegen des distalen Endbereichs des Endoskoprohres mit Rollen zur manuellen Betätigung (im Wesentlichen Herausziehen) der Züge in Pult 2 verbinden.

Ein Nachteil der Endoskop-Einrichtung nach der SU 1 522 466 ist die unbefriedigende Funktion der Einführhilfsvorrichtung, insbesondere die Schwierigkeiten beim Einführen des Endoskoprohres 3a in die Öffnung der Dichtung 13 (vgl. Zeilen 42-53 der SU 1 522 466).

Des Weiteren sollte das Nach-Außen-Stülpen des Schlauchgebildes der Einführhilfsvorrichtung so erfolgen, dass sich das "Ausstülpende" in der Nähe des rückwärtigen Bereichs des Endstücks 6 befindet. Da bei der bekannten Einrichtung der gefaltete Teil des Schlauchgebildes unmittelbar auf dem Endoskoprohr anliegt und zudem der Innenraum des Schlauchgebildes mit Druckluft beaufschlagt wird, besteht die Gefahr, dass trotz der Beaufschlagung des inneren Endes 7 des gefalteten Teil des Schlauchgebildes durch die Feder 10 dieser Teil des Schlauchgebildes auf dem Endoskoprohr klemmt und mit zunehmendem Ausstülpen des gefalteten Teils des Schlauchgebildes im Verlaufe des Einführens des Endoskops der Distalbereich des Endoskoprohres freigelegt wird. Diese Klemmwirkung wird zudem durch die relativ lockere Struktur des gefalteten Teils des Schlauchgebildes begünstigt. Schließlich behindern auch Falten oder Unebenheiten am Außenumfang des distalen Endes des Endoskoprohres, die beim Biegen des distalen Endes entstehen, die freie Bewegung des gefalteten Teils des Schlauchgebildes der Einführhilfsvorrichtung entlang des Endoskoprohres. All dies trägt dazu bei, dass die Feder 10 unter Arbeitsdruck nicht in der Lage ist, den gefalteten Teil des Schlauchgebildes zum oder gegen das Endstück 6 zu bewegen. Außerdem dichtet das Ende 7 des Schlauchgebildes, das mit zwei Ringen verbunden ist und unter die Federwindungen gesteckt wird, den Raum 14 schlecht ab.

Ein weiterer Nachteil der bekannten Endoskope liegt darin, dass die Anzahl der Biegungen, d.h. der Umschlingungswinkel ihres distalen Endes im gebogenen Zustand durch die bestimmte Anzahl der Biegungen des Endoskoprohres bzw. dessen Umschlingungswinkel begrenzt wird. Das Ende des Endoskoprohres wird durch Drehen zweier Rollen gebogen, die jeweils mit einem Zugpaar verbunden sind. Federn, die die Züge einschließen, verlängern am distalen Ende die Kanäle an der Ringwand, wobei die Ringe durch ein Gelenk miteinander verbunden sind. Zwischen den Zugenden und dem Distalgelenkring des Mechanismus zum Biegen des distalen Endes des Endoskoprohres besteht eine Lötverbindung. Wird der Zug aus der Feder nach außen bewegt, so verringert sich der Zwischenraum zwischen den Gelenkringen, wobei der Biegeradius kleiner wird. Dabei zieht der Distalgelenkring den entgegengesetzten Zug in die Feder hinein, wobei die Zwischenräume zwischen den Ringen größer werden. Der Längenunterschied zwischen dem größeren und dem kleineren Halbkreis der Röhrchenbiegung entspricht dem Produkt aus "π" und dem Querschnitt bzw. dem Durchmesser des Endoskoprohres. Die japanischen Autoren bestätigen, dass nach Bildung von drei bis vier Schleifen eine Steuerung der Biegung des distalen Endes des Endoskoprohres blockiert wurde, die Biopsiezange allerdings weiterhin funktionbereit war. Dieser Unterschied lässt sich durch die Formel von L. Euler Q₁/Q₂=e^{u.f} erklären, wobei: "Q₁" - manuelle Kraft für das Herausziehen des Zuges; "Q₂" - Restkraft von "Q₁", die auf den Distalgelenkring oder die Beißer der Biopsiezange wirkt; dabei bedeuten: "e" - Basis des natürlichen Logarithmus; "α" - Umschlingungswinkel in rad; "f" - Reibungskoeffizient des Zuges und der Feder.

Bei festgelegten Größen von "Q₁" und "f" hängt "Q₂" von der Größe "α" ab, und "α" ist bei zwei hintereinander verbundenen Zügen des Endoskopes doppelt so groß wie bei einem Zug der Biopsiezange.

Ein dritter Nachteil der bekannten Endoskope sind die Probleme bei ihrer Anwendung. Für einen erneuten Einsatz muss das Gerät desinfiziert und sterilisiert werden. Allerdings ist die bekannte Behandlungsmethode von Endoskoprohren und ihrer Kanäle nicht nur arbeits- und zeitaufwendig, sondern auch unzuverlässig - es sind Fälle von Infizierung von Patienten mit AIDS und anderen Infektionskrankheiten bekannt.

Die Vorbereitung des Prototyp-Endoskops zum Einsatz schließt neben Desinfizierung und Sterilisation auch dessen Zusammensetzen ein. Die Zahl der zusammensetzbaren Teile des Prototyp-Endoskops erreicht 10, und das Zusammensetzen selbst dauert mindestens eine halbe Stunde. Der Umgang mit Endoskopen erfordert viel Übung. Üblicherweise hält die linke Hand das Steuerpult, drückt auf die Knöpfe und dreht die Hebel, die das innere Ende des Röhrchens biegen und fixieren, während die rechte Hand das Endoskoprohr in den Darm einführt. Wenn diese Belastung für die linke Hand zu groß wird, so muß der Arzt das Einführen des Röhrchens in den Darm einstellen und die entsprechenden Hebel mit der rechten Hand drehen.

Ausgehend von diesem Stand der Technik liegt der Erfindung die Aufgabe zugrunde, ein Endoskop bzw. eine Einführhilfsvorrichtung für ein Endoskop zu schaffen, welches eine hohe Zuverlässigkeit und Sicherheit beim Einführen des Endoskoprohres in den zu untersuchenden Kanal sowie eine einfache Anwendbarkeit gewährleistet.

Die Erfindung löst diese Aufgabe mit den Merkmalen der Ansprüche 1, 13 und 16. Weitere Ausführungsformen ergeben sich aus den Unteransprüchen.

Die erfindungsgemäße Einführhilfsvorrichtung bietet den Vorteil, dass das gefaltete Schlauchgebilde im Ausgangszustand zunächst in einem Aufnahmeelement aufgenommen und gegen Beschädigung und Verunreinigungen bzw. jegliche Kontamination geschützt ist.

Nach einer bevorzugten Ausführungsform ist zwischen der Innenwandung eines das Schlauchgebilde im gefalteten Ausgangszustand bildenden Hohlzylinders und der Außenwandung des Endoskops ein Spalt vorgesehen, so dass die Verschiebebewegung des Hohlzylinders in Richtung auf das distale Ende des Endoskoprohres nicht behindert ist. Damit wird ein Freilegen des distalen Endes des Endoskoprohres und dessen Berührung mit der Innenwandung des zu untersuchenden Kanals vermieden.

Nach einer weiteren Ausführungsform besteht das Schlauchgebilde im gefalteten Zustand aus gequetschten und längs und quer verdichteten Lagen unterschiedlicher Form und Richtung. Hierdurch wird zum einen eine ausreichende Festigkeit des Hohlzylinders erreicht, um den vorstehend erläuterten Spalt zwischen Zylinder und Endoskoprohr gewährleisten zu können. Zum anderen wird durch die unterschiedliche Form und Richtung der Lagen ein Verkleben über längere Flächen vermieden, wodurch das gleichmäßige Abziehen des betreffenden Endes des Schlauchgebildes für das Nach-Außen-Stülpen erleichtert wird.

Nach einer Ausführungsform weist der Hohlzylinder des Schlauchgebildes vorzugsweise periodische Verengungen des äußeren Durchmessers und Verbreiterungen des inneren Durchmessers auf.

Nach einer bevorzugten Ausführungsform wird das rückwärtige Ende des Schlauchgebildes von einem Mechanismus für das Nach-Vorne-Schieben des Hohlzylinders des Schlauchgebildes beaufschlagt. Dieser kann im einfachsten Fall aus einer Feder bestehen (Anspruch 9) oder aus einer aktiv ansteuerbaren Vorrichtung, die einen Elektromotor (Anspruch 7) oder eine pneumatische oder hydraulische Zylindereinheit (Anspruch 8) aufweisen kann.

Nach einer Ausführungsform der Erfindung können die gesamte Einführhilfsvorrichtung oder wesentliche Komponenten davon (Schlauchgebilde; Schlauchgebilde mit Aufnahmeelement, ggf. in Verbindung mit dem Mechanismus für das Nach-Vorne-Schieben des Hohlzylinders des Schlauchgebildes) als Einwegpatrone ausgeführt sein. Hierdurch ergibt sich eine sehr einfache Handhabbarkeit der gesamten Vorrichtung.

Auf dem Endoskoprohr selbst können ein Proximalpräservativ und/oder ein Distalpräservativ vorgesehen sein, welche eine Kontamination der unmittelbaren Außenwandung des Endoskoprohres vermeiden. Das Distalpräservativ kann dabei mit einem Endstück des Endoskoprohres verbunden sein.

Die spezielle Ausbildung der Betätigungseinrichtungen für das gesteuert biegbare distale Ende des Endoskoprohres mit druckdichten in ihrer Länge dehnbaren Röhrchen, welche die eigentlichen Züge für die Betätigung des biegbaren Endes umgeben, gewährleistet geringere Betätigungskräfte bzw. ermöglicht erst ein einwandfreies Betätigen des Endes, auch wenn das Endoskoprohr mehrere Biegungen mit einem relativ großen Umschlingungswinkel durchläuft.

Nach einer Ausführungsform kann ein derartiges dehnbares Röhrchen aus einer Schraubenfeder bestehen, die in einer druckdichten flexiblen schlauchförmigen Hülle vorgesehen ist. Um bei Druckerhöhungen bzw. -senkungen die entstehenden Dehnungs- bzw. Kontraktionskräfte auf die Feder zu übertragen, muss die Hülle zumindest abschnittsweise mit der Feder gekoppelt sein, beispielsweise mittels eines um die Hülle und die Feder gewundenen Fadens.

Die Röhrchen können durch ein darin befindliches Medium, vorzugsweise eine Flüssigkeit, zusätzlich zu den eigentlichen Zügen Kräfte zur Betätigung des biegbaren Endes des Endoskops übertragen. Der erforderliche Über- bzw. Unterdruck kann vorzugsweise durch Kolben-/Zylindereinheiten erzeugt werden. Diese können entweder in einem proximalen Steuerpult des Endoskops selbst oder losgelöst davon in einer separaten Steuereinheit vorgesehen sein.

Nach einer Ausführungsform können die Züge selbst ebenfalls mit den Kolben der Kolben-/Zylindereinheiten verbunden sein. Hierdurch ergibt sich eine sehr einfache konstruktive Lösung, insbesondere für Steuerpulte, die unmittelbar am Endoskoprohr vorgesehen sind.

Die Erfindung wird nachfolgend anhand in der Zeichnung dargestellter Ausführungsbeispiele näher erläutert. In der Zeichnung zeigen:

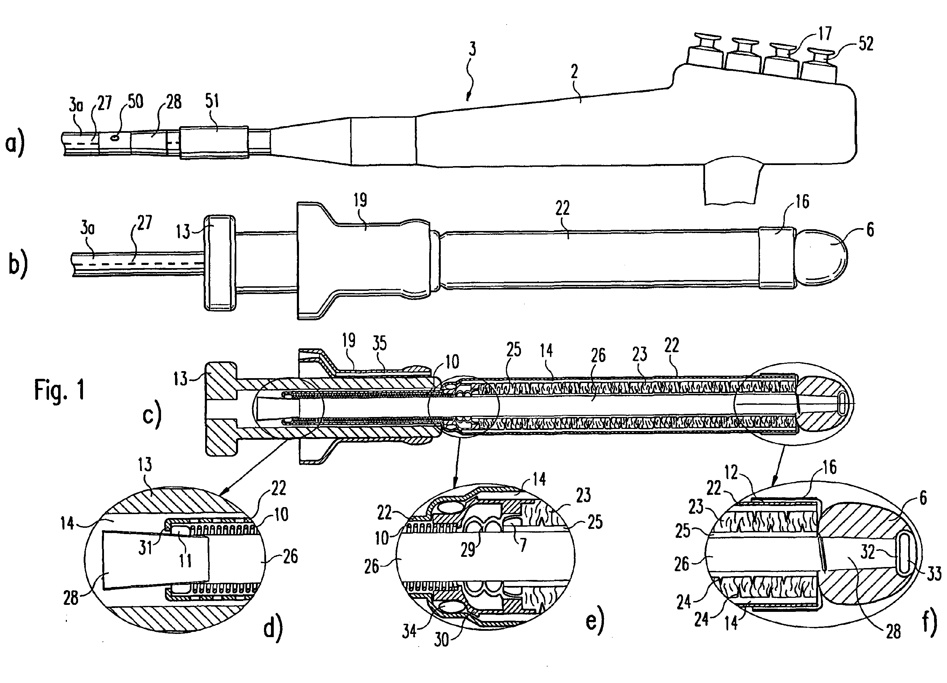

- Fig. 1: ein Endoskop nach der Erfindung mit einer Einwegpatrone zur Invagination (Fig. 1a: Seitenansicht des Steuerungspultes des Endoskops in Form eines Handgriffes; Fig. 1b: Distalteil des Endoskops mit angebrachter Einwegpatrone zur Invagination; Fig. 1c: Querschnitt der Einwegpatrone; Fig. 1d, 1e, 1f: vergrößerte Ausschnitte aus Fig. 1c);

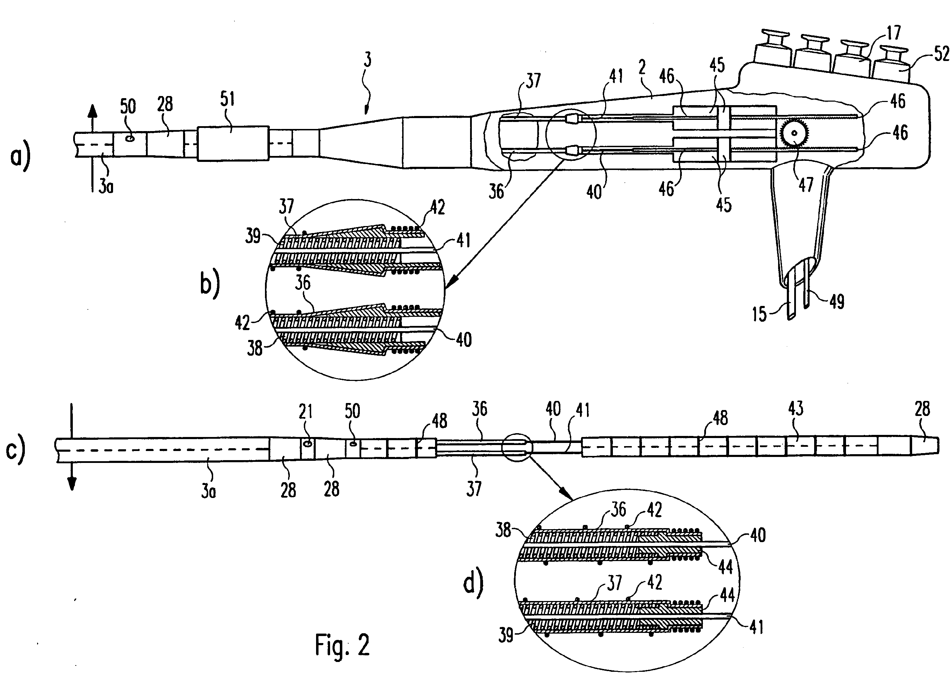

- Fig. 2: eine schematische Darstellung der Konstruktion der pneumatischen und/oder hydraulischen Betätigunsvorrichtung mit Manualantrieb bei gerader Lage des distalen Endes des Endoskoprohres (Fig. 2a: Zustand der im Steuerpult vorgesehenen Systemelemente; Fig. 2b: vergrößerter Ausschnitt eines Teilbereichs von Fig. 2a; Fig. 2c: Distalteil des endoskopischen Röhrchens mit "freigelegten" Systemelementen, wobei die senkrechten Pfeile den oberen bzw. unteren Teil des Endoskops bezeichnen; Fig. 2d: vergrößerter Ausschnitt der Fig. 2c);

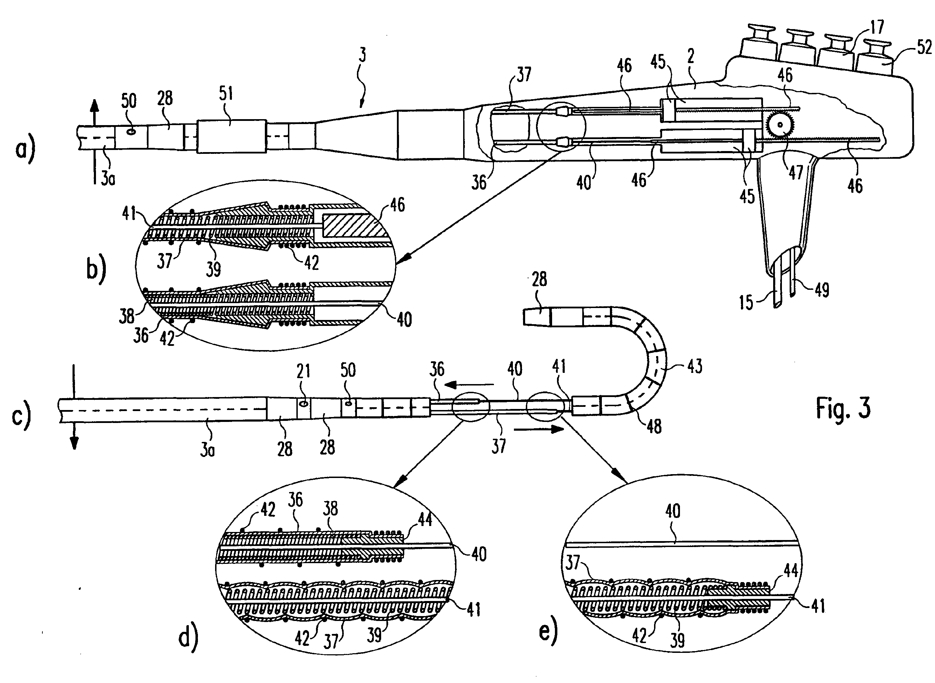

- Fig. 3: eine schematische Darstellung des Zustandes des Zugextraktoren- /-intraktorensystems bei nach unten gebogenem Ende des Endoskoprohres (Fig. 3a: Zustand der Systemelemente innerhalb des Steuerpultes; Fig. 3b: vergrößerter Ausschnitt aus Fig. 3a; Fig. 3c: Distalteil des Endoskoprohrs mit "freigelegten" Systemelementen, wobei die waagerechten Pfeile die Bewegungsrichtung der Züge andeuten; Fig. 3d und Fig. 3e: vergrößerte Ausschnitte aus Fig. 3c);

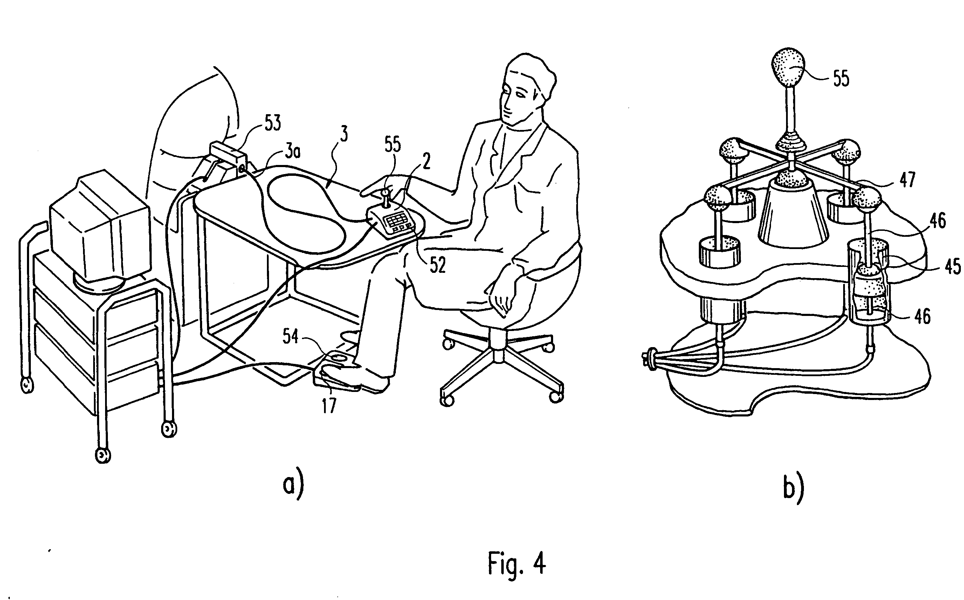

- Fig. 4a: eine Allgemeinansicht einer diagnostischen Kolonoskop-Einrichtung mit einem Mechanismus zur Bereitstellung des Endoskops, einem Tischsteuerpult und Pedalen;
- Fig. 4b: einen Aufbau eines Elementes für die synchrone Erzeugung von Unterdruck im Hohlraum der extrahierenden Züge und von Überdruck im Hohlraum der intrahierenden Züge in Form eines Kreuzes mit einem Hebel.

Die Zuordnung der Bezugszeichen in den die Erfindung darstellenden Fig. 1 bis 4 entspricht der Zuordnung der Bezugszeichen der den Stand der Technik repräsentierenden Fig. 5, die die Endoskop-Einrichtung gemäß der SU 1 522 466 zeigt.

Die erfindungsgemäße Endoskop-Einrichtung (Fig. 4) umfasst das eigentliche Endoskop 3 mit einer Einwegpatrone zum Einführen des Endoskoprohres 3a in einen zu untersuchenden Kanal. Das Endoskop umfasst ein Steuerpult 2 und eine Abzweigung, beispielsweise für eine Beobachtungseinrichtung oder das Einführen von Werkzeugen. Bei der Einrichtung gemäß Fig. 4 handelt es sich um die Kommunikationsabzweigung einer Beobachtungseinrichtung, welche das Signal einer Kamera zu einer Signalverarbeitungseinrichtung bzw. einem Monitor führt und gleichzeitig, beispielsweise mittels eines über die Abzweigung geführten Lichtleiters, Licht durch das Endoskoprohr bis an dessen distales Ende führt.

Im Endoskop 3 ist des Weiteren eine Vorrichtung für das gesteuerte Verbiegen des biegbar ausgebildeten distalen Endes des Endoskoprohres 3a vorgesehen. Diese Vorrichtung weist pneumatisch und/oder hydraulisch, manuell oder von einer Steuereinrichtung betätigbare Zugextraktoren/-intraktoren auf. Eine Luftleitung 15 und ein Ventil 17, das auf einem Steuerpult 2 oder auf einem Pedal angeordnet ist, verbinden eine Quelle für den Arbeitsdruck mit einer Öffnung 21 im vorderen Bereich des Endoskoprohres 3a, die sich in den Hohlraum einer Dichtung 13 öffnet, wobei dieser Hohlraum mit einem Hohlraum 14 einer Hülse 22 in Verbindung steht.

Ein Distalteil der Hülse 22 entspricht der Länge und dem Durchmesser nach einem nicht nach außen gestülpten, d.h. gefalteten Teil eines schlauchartigen Gebildes 23 der Einführhilfsvorrichtung. während ein Proximalteil der Hülse 22 der Länge und dem Durchmesser nach einer zusammengedrückten Feder 10 entspricht. Das nach außen gestülpte Ende 12 des Schlauchgebildes 23 ist mit einem Ring 16 auf der Hülse 22 befestigt, vorzugsweise im Bereich des vorderen Endes der Hülse 22.

Das Schlauchgebilde 23 bildet, wie aus Fig. 1 ersichtlich, im gefalteten Ausgangszustand einen Hohlzylinder mit im Wesentlichen konstanter Wanddicke, wobei in den Wandungen Verengungen 24 ausgebildet sein können. Der Innendurchmesser der Zylinderwandung ist so gewählt, dass nach dem Einführen des Endoskoprohres 3a ein Spalt 25 zwischen der Innenwandung des Hohlzylinders und der Außenwandung des Endoskoprohres 3a verbleibt. Das gefaltete Schlauchgebilde 23 ist vorzugsweise so flexibel ausgebildet, dass es bei einem Biegen des distalen Endes des Endoskoprohres 3a dessen Biegungen im Wesentlichen mitvollzieht und diese nicht behindert oder gar unmöglich macht. Zur Stabilisierung des Schlauchgebildes im gefalteten Zustand kann dieses an der Außen- und/oder Innenwandung mit einer stützenden Ummantelung umgeben sein. Diese muss jedoch so ausgebildet sein, dass beim Nach-Außen-Stülpen die Vorwärts-Schiebebewegung des gefalteten Schlauchgebildes 23 auf dem Endoskoprohr 3a nicht wesentlich behindert wird. Beispielsweise kann die Ummantelung als flexible Folie ausgebildet sein.

Innerhalb des gefalteten Schlauchgebildes kann ein Distalpräservativ 26 vorgesehen sein, in welches der distale Endbereich des Endoskoprohres 3a eingeschoben werden kann. An den Enden eines Distalpräservatives 26 und eines Proximalpräservatives 27, das zusätzlich auf das Endoskoprohr 3a aufgezogen sein kann, und an ihnen entsprechenden Stellen des Endoskoprohres 3a befinden sich Bereiche 28 zum gegenseitigen Befestigen und hermetischen Abdichten.

Eine bewegliche bzw. flexible Dichtung 29 am rückwärtigen Ende 7 des Zylinderelements zur Aufnahme des Schlauchgebildes 23 teilt den Hohlraum 14 zwischen der Innenwandung der Hülse 22 und dem darin eingesetzten gefalteten Schlauchgebilde 23 von dem Hohlraum 25, der in Verbindung mit dem Hohlraum des zu untersuchenden Kanals steht.

Ein Abstandsstück 30 schließt eine Deformation der flexiblen Dichtung 29 durch die Feder 10 aus. Die Enden der vorgespannten Feder 10 beaufschlagen das Abstandsstück 30 und einen Anschlag 11, der als Anschlagring ausgeführt sein kann, am Ende 28 des Präservatives 26. Der Anschlag 11 seinerseits stützt sich an einem Vorsprung 31 am rückwärtigen Ende der Hülse 22 ab.

Am Distalende des distalen Präservatives 26 ist ein Endstück 6 vorgesehen, das über Kanäle 32 zur Säuberung eines Schutzglases 33 und zur Luftzufuhr in den Darmtrakt verfügt, und außerdem Vorrichtungen zur mechanischen Befestigung des Endstückes 6 an der Spitze des Endoskoprohres 3a. An der Grenze zwischen den Teilen der Hülse 22 mit geringerem und größerem Durchmesser befindet sich ein Bereich mit einem Zwischendurchmesser, in den der rückwärtige Bereich des Abstandsstücks 30 hineinragt, an welchem das vordere Ende der Feder 10 angreift. Zwischen dem Außenumfang des rückwärtigen Bereichs des Abstandsstücks 30 und der Innenwandung des Bereichs der Hülse 22 mit dem Zwischendurchmesser ist ein elastischer Ring 34 vorgesehen, der den Sitz der Feder 10 im Abstandsstück 30 fixiert.

Ein Kanal 35 eines Analdilatators 19 ist zur Dekompression des Darmtraktes während der Intubation vorgesehen.

Im Endoskoprohr 3a bzw. im Steuerpult 2 sind elastische Röhrchen 36, 37, Endfedern 38, 39 und Züge 40, 41 vorgesehen. Die Röhrchen 36, 37 sind an den Federn 38, 39 durch einen Faden 42 fixiert. In der Nähe eines Mechanismus 43 zum Biegen des distalen Endes des Endoskoprohres 3a sind die Enden der Röhrchen 36, 37 mit Pfropfen 44 verschlossen, die außerdem die Federn 38, 39 mit den Zügen 40, 41 verbinden. Die Außenenden der Röhrchen 36, 37 sind hermetisch mit Überdruck- und Unterdruckquellen 45 verbunden. Die Außenenden der Züge 40, 41 sind mit ihren manuellen Betätigungselementen 46 verbunden, und diese mit einem Element 47, das für die synchrone Erzeugung von Unterdruck im Hohlraum des extrahierenden Zuges 40 und von Überdruck im Hohlraum des intrahierenden Zuges 41 sorgt.

Das Endoskop 3 weist an seinem distalen Ende des Endoskoprohres 3a Elemente 48 in dessen äußerer Ummantelung auf, welche mittels des Mechanismus 43 ein gesteuertes Biegen des distalen Ende ermöglichen.

Des Weiteren weist das Endoskop 3 eine Luftleitung 49 auf, welche mit zwei Öffnungen 50 zur Unterdruckfixierung der Präservative 26, 27 am Endoskoprohr 3a verbunden ist, wobei auf dem Steuerpult 2 ein Ventil oder Hahn 52 für die Luftleitung 49 vorgesehen ist.

Auf dem proximalen Ende des Endoskoprohres 3a ist eine abnehmbare Manschette 51 vorgesehen.

Wie aus Fig. 4 ersichtlich, wird bei der darin dargestellen Ausführungsform einer Endoskop-Einrichtung nach der Erfindung die Dichtung 13 in einem abnehmbaren Mechanismus 53 zur Bereitstellung des Endoskoprohres 3a gehalten. Mit einem Pedal 54 wird ein Elektromotor des Mechanismus 53 eingeschaltet, und durch einen Hebel 55 wird gleichzeitig das innere Ende des Röhrchens 3 gebogen.

Als Orientierung zum richtigen Zusammensetzen des Präservatives 27 und des Endoskoprohres 3a dienen punktierte Linien, die auf ihnen angebracht sind. Nach dem Zusammensetzen wird auf das Endoskoprohr 3a die Patrone aufgesetzt und mit dem Endstück 6 verbunden. Nach Verschiebung der Dichtung 13 nach links (Fig. 1) und nach Erhalt des Zuganges zum Proximalende 28 des Präservatives 26 wird dieses an dem entsprechenden Bereich 28 des Röhrchens 3 befestigt. Durch Druck auf den Hahn 52 werden die Präservative 26, 27 durch Unterdruck an dem Endoskoprohr 3a fixiert. Am Ende der Vorbereitung des Endoskops zum Einsatz wird die Dichtung 13 mit dem Dilatator 19 auf der Hülse 22 in die Ausgangslage zurückgebracht.

Nachdem der Patient hingelegt wurde, wird die Patrone eingeschmiert und in den Enddarm eingeführt. Anschließend (nach Kontrolle der Ampulle) wird der Bereitstellungsmechanismus 53 mit der Dichtung 13 zusammengesetzt. Durch Druck auf den Hahn 17 wird in dem Hohlraum 14 Überdruck erzeugt, durch den das Abstandsstück 30 von der Verkupplung mit der Fixierung 34 und der Hülse 22 freigegeben wird, wodurch die Feder 10 in Arbeitsstellung gebracht wird. Das Herausdrehen des Invaginators 23 und das Einführen des Röhrchens 3 in den Grimmdarm geschieht während des Drückens auf das Pedal 54 unter Arbeitsdruck im Hohlraum 14. Während der Endoskopie muss der Darm aufgeblasen sein. Das Gas tritt ständig in den Darmtrakt über den Gas-/Flüssigkeitskanal des Röhrchens 3 sowie über den Kanal 32 des Endstückes 6, wodurch auch das Eindringen des Darminhaltes unter das Schutzglas 33 verhindert wird. Die Evakuation des Gases aus dem Darmtrakt erfolgt über den Kanal 35 des Analdilatators 19.

Das Einführen des Endoskoprohres 3a in den zu untersuchenden Kanal erfolgt in der Weise, dass der Anschlagring 11 von einem konischen Bereich des Endoskoprohres oder einem darauf fixierten weiteren Anschlag beaufschlagt und demzufolge die Feder 10, sowie das gefaltete Schlauchgebilde 23 mitgenommen wird. Hierdurch erfolgt ein Herausziehen und Nach-Außen-Stülpen des gefalteten Teils des Schlauchgebildes 23, wobei sich der nach außen gestülpte Teil an die Innenwandung des zu untersuchenden Kanals anlegt. Durch die Beaufschlagung des Schlauchgebildes 23 mittels der Feder 10 wird dieses gegen das Endstück bewegt, so dass das Ausstülpende sich immer am rückwärtigen Ende des Endstücks 6 befindet. Hierdurch wird ein Freilegen des distalen Endes des Endoskoprohres 3a und dessen Kontakt mit der Innenwandung des zu untersuchenden Kanals vermieden.

Das Biegen des Mechanismus 43 erfolgt mit Hilfe von Quellen 45 von Überdruck und Unterdruck, von manuellen Betätigungselementen 46 der Züge 40, 41 und mit Hilfe von Vorrichtungen 47, die für die synchrone Erzeugung von Unterdruck im Hohlraum des Röhrchens 36, das den in Fig. 3 mit Zugkraft beaufschlagten extrahierenden Zug 40 einschließt, und von Überdruck im Hohlraum des Röhrchens 37, das den intrahierenden Zug 41 einschließt, sorgen.

Unter Einwirkung von Unterdruck verkürzt sich das elastische Röhrchen 36 und die Feder 38. Da ihr proximales Ende festgelegt ist und das distale Ende mit dem Zug 40 verbunden ist, erleichtert diese Verkürzung die manuelle Extraktion des Zuges 40.

Der Überdruck im Röhrchen 37 verlängert dieses und die Feder 39 zum Mechanismus 43 hin, und damit wird die Intraktion des Zuges 41 erleichtert. Der um die Röhrchen 36, 37 gewickelte Faden 42 verbindet diese mit den Federn 38, 39.

Somit werden durch Überdruck und durch Unterdruck die Röhrchen 36, 37 und die Federn 38, 39 verkürzt bzw. verlängert. Dadurch wirkt eine Kraft auf die inneren Enden der Züge 40 und 41; die manuelle Extraktion/Intraktion der Züge 40, 41 bewirkt eine synchrone Krafteinwirkung auf ihre äußeren Enden.

Durch das oben beschriebene Verfahren biegt sich der Mechanismus 43 des Endoskoprohres 3a nach oben (Fig. 3). Beim Biegen des Mechanismus 43 nach oben bewegen sich alle oben beschriebenen Elemente in entgegengesetzten Richtungen. Die Bewegung des Mechanismus 43 nach links oder rechts wird durch das zweite Zugpaar realisiert, das analog arbeitet. Bei Zwischenrichtungen wird der Mechanismus mit Hilfe beider Zugpaare gebogen, wobei diese bei einer manuellen Betätigung mittels zweier Betätigungsräder am Steuerpult 2 üblicherweise nacheinander betätigt werden. Die in Fig. 4b dargestellte Steuereinrichtung, bei der die Vorrichtung 47 in Form eines Kreuzes mit einem Betätigungshebel 55 ausgebildet ist, ermöglicht ein gleichzeitiges Biegen des Mechanismus 43 in jede Richtung.

Da während der Kolonoskopie das Endoskop alle natürlichen Windungen des Dickdarmes nachvollzieht, sollte seine Extubation nicht forciert werden. Der Analdilatator 19, durch den die Extubation durchgeführt werden soll, verringert die unangenehmen Empfindungen im Verlaufe dieses Prozesses auf ein Minimum.

Eine praktisch bedeutende Variante der Anwendung des vorgeschlagenen Endoskops ist ein diagnostisches Kolonoskop mit einem Endoskoprohr ohne Biopsiekanal. Die Einwegpatrone zur Invagination des Endoskoprohres ermöglicht einen einfachen atraumatischen Transport des Endoskoprohres 3a im Dickdarm, und die Einrichtung für das gesteuerte Biegen des distalen Endes des Endoskoprohres ermöglicht das Biegen des inneren Endes des Endoskoprohres 3a derart, dass die natürliche Achse des Dickdarmes bzw. dessen Windungen nachvollzogen werden. Die Präservative schützen den Patienten vor einer Infektion, die sich im Endoskoprohr einnistet, da das Endoskoprohr während der Endoskopie nicht unmittelbar mit dem zu untersuchenden Kanal in Berührung kommt. Durch die Steuerungsergonomie des diagnostischen Kolonoskopes ist dieses auch einfach zu bedienen: Während der Endoskopie sitzt der Arzt auf einem Stuhl und sieht auf einen Bildschirm. Mit dem Fuß schaltet er den Hahn 17 ein und bedient danach den Schalter 54. Mit der rechten Hand bedient er den Hebel 55, mit der linken Hand drückt er je nach Bedarf auf den Hahn und säubert damit das Schutzglas 33. Ein derartiges Kolonoskop benötigen vor allem Hausärzte, Gastroenterologen und Chirurgen zum regelmäßigen Screening nach Dickdarmkrebs. Nach einer ersten Voruntersuchung werden die "verdächtigen" Patienten danach zur eingehenderen Untersuchung, zur Durchführung von Biopsie, zur Entfernung von Polypen und, falls notwendig, zur Durchführung einer Operation an Spezialkliniken bzw. Spezialisten überwiesen.

### Bezugszeichenliste

- 1 -: Okular
- 2 -: Steuerpult
- 3 -: Endoskop
- 3a: Endoskoprohr
- 4 -: nach außen gestülptes Teil des Schlauchgebildes
- 5 -: Quelle des Arbeitsdruckes im Hohlraum 14
- 6 -: Endstück des Endoskoprohres 3a
- 7 -: nicht nach außen gestülptes Teil des Schlauchgebildes 23
- 8,9 -: Ringe am Ende 7 des Schlauchgebildes 23
- 10 -: Feder
- 11 -: Anschlagring für die Feder 10
- 12 -: umgestülptes Ende des Schlauchgebildes 23
- 13 -: äußere bewegliche Dichtung des Endoskoprohres 3a
- 14 -: Hohlraum
- 15 -: Luftleitung zur Einleitung des Arbeitsdruckes in den Hohlraum 14
- 16 -: Ring zur Fixierung des Endes 12 des Schlauchgebildes 23
- 17 -: Ventil für die Luftleitung 15
- 18 -: Manometer
- 19 -: Analdilatator
- 20 -: Enddarm
- 21 -: Öffnung der Luftleitung 15 am Endoskoprohr 3a
- 22 -: Hülse der Patrone zur Erleichterung des Einführens
- 23 -: Schlauchgebilde
- 24 -: Verengungen und Verbreiterungen des gefalteten Schlauchgebildes 23
- 25 -: Spalt (Hohlraum) zwischen dem Trägerteil des Schlauchgebildes 23 und dem Präservativ 26 bzw. dem Endoskoprohr
- 26 -: Distalpräservativ für das Endoskoprohr 3a
- 27 -: Proximalpräservativ für das Endoskoprohr 3a
- 28 -: Bereiche auf dem Röhrchen 3 und auf den Enden der Präservative 26, 27 zu ihrer hermetischen Verbindung
- 29 -: innere bewegliche Dichtung zwischen dem Endoskoprohr 3a und dem Ende 7 des Trägerteils des Schlauchgebildes 23
- 30 -: Abstandsstück zwischen der Feder 10 und dem Schlauchgebilde 23
- 31 -: Vorsprung auf der Hülse 22 für die Stütze 11
- 32 -: Kanal im Endstück 6
- 33 -: Schutzglas des Endstückes 6
- 34 -: elastischer Ring zur Fixierung der Feder 10 im zusammengedrückten Zustand
- 35 -: Kanal im Analdilatator 19
- 36 -: unteres elastisches Röhrchen der Betätigungsvorrichtung
- 37 -: oberes elastisches Röhrchen der Betätigungsvorrichtung
- 38 -: untere Feder der Betätigungsvorrichtung
- 39 -: obere Feder der Betätigungsvorrichtung
- 40 -: unterer Zug der Betätigungsvorrichtung
- 41 -: oberer Zug der Betätigungsvorrichtung
- 42 -: Faden zur Fixierung der elastischen Röhrchen 36, 37 auf den Federn 38, 39
- 43 -: Mechanismus zum Biegen des distalen Endes des Endoskoprohres 3a
- 44 -: Pfropfen zum Verschluss der Röhrchen 36, 37 und zum Verbinden der Federn 38, 39 mit den Zügen 40, 41
- 45 -: Überdruck- und Unterdruckquellen
- 46 -: manuelle Betätigungsvorrichtungen für die Züge 40, 41
- 47 -: Vorrichtung für die synchrone Erzeugung von Unterdruck im Hohlraum des manuell extrahierenden Zuges und von Überdruck im Hohlraum des intrahierenden Zuges
- 48 -: Elemente in der äußeren Ummantelung des Endoskoprohres 3a, die das gesteuerte Biegen des distalen Endes ermöglichen
- 49 -: Luftleitung für das Fixieren der Präservative 26, 27
- 50 -: Distal- und Proximalöffnungen der Luftleitung 49 auf dem Endoskoprohr 3a
- 51 -: Manschette
- 52 -: Ventil für die Luftleitung 49 auf dem Steuerpult 2
- 53 -: Mechanismus zur Bereitstellung des Endoskoprohres 3a
- 54 -: Pedal zum Einschalten des Motors des Mechanismus 54
- 55 -: Hebel der Vorrichtung 47 in Form eines Kreuzes

## Patentansprüche

1. Einführhilfsvorrichtung für ein Endoskop
a) welche am distalen Ende des Endoskoprohres (3a) anordenbar ist und nach dem Prinzip des sich ausstülpenden Schlauchgebildes (23) arbeitet,
**dadurch gekennzeichnet,**
b) **dass** die Einführhilfsvorrichtung ein Aufnahmeelement (22) für das Schlauchgebilde (23) in einem zusammengefalteten Ausgangszustand aufweist, wobei das vordere Ende des Schlauchgebildes mit dem Aufnahmeelement verbunden ist und
c) **dass** der gefaltete Teil des Schlauchgebildes (23) am Außen- und/oder Innenumfang von einer flexiblen Hülle umgeben ist.

2. Einführhilfsvorrichtung nach Anspruch 1, **dadurch gekennzeichnet, dass** das Schlauchgebilde (23) im gefalteten Ausgangszustand einen ausreichend stabilen Hohlzylinder derart bildet, dass zwischen der Innenwandung des Hohlzylinders und der Außenwandung des das Schlauchgebilde (23) durchgreifenden Endoskoprohres (3a) auch bei einer Druckbeaufschlagung des Schlauchgebildes (23) eine Verschiebebewegung des Hohlzylinders in Richtung auf das distale Ende des Endoskoprohres ermöglicht ist.

3. Einführhilfsvorrichtung nach Anspruch 1 oder 2, **dadurch gekennzeichnet, dass** das Schlauchgebilde (23) im gefalteten Ausgangszustand aus gequetschten und längs und quer verdichteten kurzen Lagen unterschiedlicher Form geformt ist.

4. Einführhilfsvorrichtung nach Anspruch 3, **dadurch gekennzeichnet, dass** die Lagen in unterschiedlichen Winkeln zur Längsachse des Endoskoprohres (3a) angeordnet sind.

5. Einführhilfsvorrichtung nach einem der vorhergehenden Ansprüche, **dadurch gekennzeichnet, dass** das Schlauchgebilde (23) an seinem rückwärtigen Ende in einem Trägerteil aufgenommen ist.

6. Einführhilfsvorrichtung nach einem der vorhergehenden Ansprüche, **dadurch gekennzeichnet, dass** das rückwärtige Ende des Hohlzylinders mittelbar oder unmittelbar von einen Mechanismus für das Nach-Vorne-Schieben des Hohlzylinders beaufschlagt ist.

7. Einführhilfsvorrichtung nach Anspruch 6, **dadurch gekennzeichnet, dass** der Mechanismus für das Nach-Vorne-Schieben des Hohlzylinders des Schlauchgebildes einen Elektromotor umfasst, gesteuert oder geregelt den Hohlzylinder der Schlauchgebildes 23 nach vorne bewegt.

8. Einführhilfsvorrichtung nach Anspruch 6, **dadurch gekennzeichnet, dass** der Mechanismus für das Nach-Vorne-Schieben des Hohlzylinders des Schlauchgebildes eine Kolben-/Zylindereinheit umfasst, welche bei Beaufschlagung mit Arbeitsdruck den Hohlzylinder des Schlauchgebildes 23 nach vorne bewegt, wobei der Kolben vorzugsweise vom Endoskoprohr (3a) durchgriffen wird.

9. Einführhilfsvorrichtung nach Anspruch 6, **dadurch gekennzeichnet, dass** der Mechanismus für das Nach-Vorne-Schieben des Hohlzylinders des Schlauchgebildes eine vorgespannte Feder (10) umfasst, welche den Hohlzylinder zumindest während des Ausstülpvorgangs an seinem rückwärtigen Ende beaufschlagt.

10. Einführhilfsvorrichtung nach Anspruch 9, **dadurch gekennzeichnet, dass** die Beaufschlagung des Schlauchgebildes (23) durch die Feder (10), vorzugsweise durch eine Beaufschlagung des Trägerteils, mittels einer ansteuerbaren Vorrichtung auslösbar ist, vorzugsweise durch den den Innenraum (14) des Schlauchgebildes beaufschlagenden Arbeitsdruck.

11. Einführhilfsvorrichtung nach einem der vorhergehenden Ansprüche, **dadurch gekennzeichnet, dass** die Einführhilfsvorrichtung als auf das distale Ende des Endoskoprohres aufsetzbare und damit verbindbare Einwegpatrone ausgebildet ist, welche zumindest das als Hülse ausgebildete Aufnahmeelement (22) und das darin vorgesehene Schlauchgebilde (23) umfasst.

12. Einführhilfsvorrichtung nach Anspruch 11, **dadurch gekennzeichnet, dass** die Einwegpatrone zusätzlich die Feder (10) sowie die Vorrichtung zur Auslösung der Federbeaufschlagung des Schlauchgebildes (23) umfasst.

13. Schlauchgebilde für eine Einführhilfsvorrichtung nach einem der vorhergehenden Ansprüche, **dadurch gekennzeichnet, dass** das Schlauchgebilde (23) im gefalteten Ausgangszustand einen Hohlzylinder bildet und dass der gefaltete Teil des Schlauchgebildes (23) am Außen- und/oder Innenumfang von einer flexiblen Hülle umgeben ist.

14. Schlauchgebilde nach Anspruch 13, **dadurch gekennzeichnet, dass** das Schlauchgebilde (23) im gefalteten Ausgangszustand aus gequetschten und längs und quer verdichteten kurzen Lagen unterschiedlicher Form geformt ist, wobei die Lagen vorzugsweise in unterschiedlichen Winkeln zur Längsachse des endoskopischen Röhrchens angeordnet sind.

15. Schlauchgebilde nach einem der Ansprüche 13 oder 14, **dadurch gekennzeichnet, dass** das Schlauchgebilde (23) an seinem rückwärtigen Ende in einem Trägerteil aufgenommen ist.

16. Endoskop, insbesondere Kolonoskop, mit einer Einführhilfsvorrichtung nach einem der Ansprüche 1 bis 12.

## Claims

1. Insertion aid for an endoscope
a) which can be disposed at the distal end of the endoscope tube (3a) and works according to the principle of the evaginating hose formation (23),
**characterised in**
b) **that** the insertion aid has a holding element (22) for the hose formation (23) in a folded-up initial state, wherein the front end of the hose formation is connected to the holding element, and
c) **that** the folded part of the hose formation (23) is surrounded by a flexible sheath at the outer and/or inner circumference.

2. Insertion aid according to Claim 1, **characterised in that** the hose formation (23) forms a sufficiently stable hollow cylinder in the folded initial state such that the hollow cylinder can also be displaced in the direction of the distal end of the endoscope tube between the inner wall of the hollow cylinder and the outer wall of the endoscope tube (3a) passing through the hose formation (23) when the hose formation (23) is pressurised.

3. Insertion aid according to Claim 1 or 2, **characterised in that** the hose formation (23) is shaped from squeezed and longitudinally and transversely compressed short layers of differing form in the folded initial state.

4. Insertion aid according to Claim 3, **characterised in that** the layers are disposed at differing angles to the longitudinal axis of the endoscope tube (3a).

5. Insertion aid according to any one of the preceding Claims, **characterised in that** the hose formation (23) is held in a carrier part at its rear end.

6. Insertion aid according to any one of the preceding Claims, **characterised in that** the rear end of the hollow cylinder is indirectly or directly acted upon by a mechanism for the forward displacement of the hollow cylinder.

7. Insertion aid according to Claim 6, **characterised in that** the mechanism for the forward displacement of the hollow cylinder of the hose formation comprises an electric motor and, controlled or regulated, moves the hollow cylinder of the hose formation (23) forwards.

8. Insertion aid according to Claim 6, **characterised in that** the mechanism for the forward displacement of the hollow cylinder of the hose formation comprises a piston-cylinder unit which, when acted upon by working pressure, moves the hollow cylinder of the hose formation (23) forwards, wherein the endoscope tube (3a) preferably passes through the piston.

9. Insertion aid according to Claim 6, **characterised in that** the mechanism for the forward displacement of the hollow cylinder of the hose formation comprises a biased spring (10) which acts upon the hollow cylinder at least during the evagination process at its rear end.

10. Insertion aid according to Claim 9, **characterised in that** the action upon the hose formation (23) by the spring (10), preferably by acting upon the carrier part, can be initiated by means of a controllable device, preferably by the working pressure acting upon the interior (14) of the hose formation.

11. Insertion aid according to any one of the preceding Claims, **characterised in that** the insertion aid is formed as a disposable cartridge which can be placed on the distal end of the endoscope and connected thereto and which comprises at least the holding element (22), formed as a sleeve, and the hose formation (23) provided therein.

12. Insertion aid according to Claim 11, **characterised in that** the disposable cartridge additionally comprises the spring (10) as well as the device for initiating the spring action on the hose formation (23).

13. Hose formation for an insertion aid according to any one of the preceding Claims, **characterised in that** the hose formation (23) forms a hollow cylinder in the folded initial state, and that the folded part of the hose formation (23) is surrounded by a flexible sheath at the outer and/or inner circumference.

14. Hose formation according to Claim 13, **characterised in that** the hose formation (23) is shaped from squeezed and longitudinally and transversely compressed short layers of differing form in the folded initial state, wherein the layers are preferably disposed at differing angles to the longitudinal axis of the small endoscopic tube.

15. Hose formation according to any one of Claims 13 and 14, **characterised in that** the hose formation (23) is held in a carrier part at its rear end.

16. Endoscope, in particular colonoscope, with an insertion aid according to any one of Claims 1 to 12.

## Revendications

1. Dispositif d'aide à l'insertion endoscopique
a) qui peut être disposé à l'extrémité distale du tube endoscopique (3a) et fonctionne selon le principe d'une structure de tuyau (23) qui se gonfle,
**caractérisé en ce que**
b) le dispositif d'aide à l'introduction présente un élément de réception (22) pour la structure de tuyau (23) dans un état initial replié, moyennant quoi l'extrémité avant de la structure de tuyau est reliée à l'élément de réception, et
c) la partie repliée de la structure de tuyau (23) est entourée, au niveau de la périphérie extérieure et / ou intérieure, par une gaine flexible.

2. Dispositif d'aide à l'insertion selon la revendication 1, **caractérisé en ce que** la structure de tuyau (23), dans l'état initial replié, forme un cylindre creux suffisamment stable, de telle sorte que, entre la paroi intérieure du cylindre creux et la paroi extérieure du tube endoscopique (3a) pénétrant dans la structure de tuyau (23), même pendant une mise sous pression de la structure de tuyau (23), un déplacement par coulissement du cylindre creux en direction de l'extrémité distale du tube endoscopique est rendu possible.

3. Dispositif d'aide à l'insertion selon l'une quelconque des revendications 1 ou 2, **caractérisé en ce que** la structure de tuyau (23), dans l'état initial replié, est constituée de couches serrées et courtes, comprimées longitudinalement et transversalement, de formes différentes.

4. Dispositif d'aide à l'insertion selon la revendication 3, **caractérisé en ce que** les couches sont disposées selon différents angles par rapport à l'axe longitudinal du tube endoscopique (3a).

5. Dispositif d'aide à l'insertion selon l'une quelconque des revendications précédentes, **caractérisé en ce que** la structure de tuyau (23) est reçue dans un élément de support au niveau de son extrémité arrière.

6. Dispositif d'aide à l'insertion selon l'une quelconque des revendications précédentes, **caractérisé en ce que** l'extrémité arrière du cylindre creux peut être sollicitée directement ou indirectement par un mécanisme permettant de pousser en avant / en arrière le cylindre creux.

7. Dispositif d'aide à l'insertion selon la revendication 6, **caractérisé en ce que** le mécanisme permettant de pousser en avant / en arrière le cylindre creux de la structure de tuyau comprend un moteur électrique, qui commande ou règle le déplacement en avant du cylindre creux de la structure de tuyau 23.

8. Dispositif d'aide à l'insertion selon la revendication 6, **caractérisé en ce que** le mécanisme permettant le déplacement en avant / en arrière du cylindre creux de la structure de tuyau comprend une unité de piston / cylindre qui, lors de la sollicitation avec une pression de service, déplace en avant le cylindre creux de la structure de tuyau 23, moyennant quoi le piston est de préférence traversé par le tube endoscopique (3a).

9. Dispositif d'aide à l'insertion selon la revendication 6, **caractérisé en ce que** le mécanisme permettant le déplacement en avant / en arrière du cylindre creux de la structure de tuyau comporte un ressort (10) précontraint, qui sollicite le cylindre creux au moins pendant le processus de gonflement au niveau de son extrémité arrière.

10. Dispositif d'aide à l'insertion selon la revendication 9, **caractérisé en ce que** la sollicitation de la structure de tuyau (23) par l'intermédiaire du ressort (10), de préférence par l'intermédiaire d'une sollicitation de l'élément de support, peut être déclenchée à l'aide d'un dispositif pouvant être excité, de préférence sous l'effet de la pression de service sollicitant l'enceinte intérieure (14) de la structure de tuyau.

11. Dispositif d'aide à l'insertion selon l'une quelconque des revendications précédentes, **caractérisé en ce que** le dispositif d'aide à l'insertion est configuré sous la forme d'un mandrin unidirectionnel pouvant être appliqué sur l'extrémité distale du tube endoscopique et relié à celle-ci, lequel comprend au moins l'élément de réception (22) configuré sous la forme d'une gaine, et la structure de tuyau (23) prévue à l'intérieur de celle-ci.

12. Dispositif d'aide à l'insertion selon la revendication 11, **caractérisé en ce que** le mandrin unidirectionnel comprend en outre le ressort (10) ainsi que le dispositif permettant de déclencher la sollicitation par le ressort de la structure de tuyau (23).

13. Structure de tuyau pour un dispositif d'aide à l'insertion selon l'une quelconque des revendications précédentes, **caractérisée en ce que** la structure de tuyau (23), dans l'état initial replié, forme un cylindre creux, et **en ce que** l'élément replié de la structure de tuyau (23) est entourée d'une gaine flexible au niveau de la périphérie extérieure et / ou intérieure.

14. Structure de tuyau selon la revendication 13, **caractérisée en ce que** la structure de tuyau (23), dans l'état initial replié, est constitué de couches serrées et courtes, comprimées longitudinalement et transversalement, de différentes formes, moyennant quoi les couches sont de préférence disposées selon des angles différents par rapport à l'axe longitudinal du petit tube endoscopique.

15. Structure de tuyau selon l'une quelconque des revendications 13 ou 14, **caractérisée en ce que** la structure de tuyau (23) est logée dans un élément de support, au niveau de son extrémité arrière.

16. Endoscope, en particulier colonoscope, comportant un dispositif d'aide à l'insertion selon l'une quelconque des revendications 1 à 12.
